# FASCICULE DE BREVET EUROPEEN

(11) **EP 0 745 371 B1**
(45) Date de publication et mention de la délivrance du brevet: **17.12.1997**
(21) Numéro de dépôt: 96401096.1
(22) Date de dépôt: 20.05.1996
(51) Int. Cl.: A61K 7/02, A61K 7/027

(54) **Composition cosmétique sous forme de pâte souple, procédé de préparation et utilisation**
Kosmetische Zusammensetzung in Form von einer weichen Paste sowie Herstellungsverfahren und Anwendung
Cosmetic composition in form of a supple paste, preparation process and utilization

(30) Priorité: 02.06.1995 FR 9506606
(43) Date de publication de la demande: 04.12.1996
(73) Titulaire: L'OREAL, 75008 Paris (FR)
(72) Inventeur: Miguel-Colombel, Dolorès, 92340 Bourg La Reine (FR); Jacques, Véronique, 92340 Bourg La Reine (FR)
(74) Mandataire: Dodin, Catherine

(56) Documents cités:
- EP-A- 0 462 709
- FR-A- 2 486 800
- US-A- 5 225 186
- MANUFACTURING CHEMIST, vol. 58, no. 8, Août 1987, LONDON GB, page 65 XP002011793 "lip product"

## Description

La présente invention a trait à une composition cosmétique se présentant sous forme d'une pâte souple, pouvant être utilisée pour le soin et/ou le maquillage des lèvres du visage et/ou de la peau. L'invention concerne également un procédé de préparation de cette composition et son utilisation.

Les compositions cosmétiques pouvant être appliquées sur la peau ou les lèvres du visage comme produit de maquillage ou de soin, telles que les bases pour lèvres ou les rouges à lèvres par exemple, contiennent généralement des corps gras dont des cires et des huiles, des pigments et/ou charges et, éventuellement, des additifs.
Il est connu que plus la quantité de cires présente dans la composition est importante, plus celle-ci a une consistance ferme, ce qui permet son utilisation sous forme de bâton. Or la présentation, en particulier d'un rouge à lèvres, sous forme de bâton présente certains inconvénients: le dessin des contours des lèvres est malaisé et la tenue du bâton à la chaleur n'est pas optimale.
Il est également connu des compositions cosmétiques se présentant sous forme de pâte souple, applicable à l'aide d'un pinceau par exemple. Ces compositions contiennent généralement une faible quantité de cires, de l'ordre de 3-8% et des corps gras, notamment de type pâteux et huileux. Les corps gras de type pâteux sont généralement présents en quantité importante, de manière à obtenir une composition de consistance et de viscosité adéquates en permettant l'application.
On a toutefois constaté que ces compositions ne permettaient pas de déposer sur la peau un film possédant de bonnes qualités sensorielles. En effet, certains corps gras pâteux présentent l'inconvénient de conférer une sensation de collant au film déposé. De plus, un autre inconvénient de ces compositions réside dans leur capacité à migrer, c'est-à-dire dans le fait qu'elles ont tendance à se propager à l'intérieur des ridules de la peau, notamment celles qui entourent les lèvres, en créant un effet inesthétique, cette migration étant en partie due à la présence en quantité importante de corps gras huileux.
On connaît ainsi, par EP462709, une composition cosmétique, notamment de rouge à lèvres comprenant 30-60% en poids d'huiles légères, 1-10% en poids de cires, 30-60% en poids d'une phase pulvérulente comprenant de la silice sphérique, des particules de Nylon et une poudre sphérique, les composés sphériques représentant 0,1 à 60% en poids de la composition finale.
On sait également, par le document Manufacturing Chemist, Août 1987, no8, page 65, qu'un agent épaississant de type 'Bentone Gel', peut être incorporé dans une composition de rouge à lèvres afin d'en améliorer la stabilité à la chaleur, ou afin de diminuer la migration des huiles, ou encore afin d'améliorer la dispersion des pigments.

La présente invention a pour but de pallier les inconvénients de l'art antérieur et propose une composition qui migre peu lorsqu'elle est appliquée sur un support, tout en donnant un film de très bonne tenue, qui ne transfère pas et ne tache pas un support avec lequel il serait en contact, et ne migre pas au cours du temps, tout en étant agréable à porter.

Un objet de la présente invention est donc une composition cosmétique se présentant sous forme de pâte souple, comprenant au moins une cire et au moins une huile épaissie par la présence d'au moins un agent épaississant et d'au moins 6% en poids de charges, ledit mélange 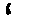 huile + charges + épaississant 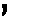 présentant une viscosité de 50-250 Pa.s, et ladite composition finale présente une viscosité dynamique à 25°C de 3-30 Pa.s.
Un autre objet de l'invention est l'utilisation de cette composition pour obtenir un film brillant et/ou de très bonne tenue et/ou qui ne transfère pas et/ou qui ne tache pas et/ou qui ne migre pas au cours du temps.
Encore un autre objet de l'invention est un procédé de préparation de la composition précédente, qui consiste à préparer un prémélange d'au moins une partie des huiles avec au moins une partie de l'agent épaississant, à homogénéiser ledit prémélange, puis à ajouter le reste des constituants.

La composition selon l'invention permet l'obtention d'un film homogène, aisément applicable et s'étalant facilement et uniformément. Le film obtenu présente également une texture légère et reste confortable à porter tout au long de la journée.
Un autre avantage de la composition selon l'invention est que, de manière inattendue, elle permet l'obtention d'un film d'aspect brillant, bien que contenant un taux important de charges, notamment sphériques, qui sont habituellement connues pour procurer un aspect mat au film.

Une des caractéristiques de la composition selon l'invention est qu'elle contient très peu, et de préférence pas, d'huiles sous forme liquide. En effet, on a constaté que le fait d'ajouter les huiles sous forme épaissie, voire gélifiée, grâce à la présence d'agent épaississant et d'une quantité importante de charges notamment sphériques, permet d'éviter, au moins en partie, le problème de migration du film, tout en conservant une application de la composition sur la peau aisée, et des qualités sensorielles adéquates. Sans être tenu par cette explication, on peut considérer que cet effet est dû, notamment, aux charges, qui, lorsqu'elles sont mélangées avec les huiles, vont absorber au moins en partie lesdites huiles et conduire à leur gélification au moins partielle. Ce caractère gélifié est également accentué par la présence d'agent épaississant dans la composition.

La composition selon la présente invention est donc une pâte souple, dont on peut mesurer la viscosité, par opposition à la structure solide d'un bâton, ou stick, dont on ne peut pas mesurer la viscosité. Ladite viscosité dynamique à 25°C est généralement comprise entre 3 et 30 Pa.s, mesurée avec un viscosimètre rotatif CONTRAVES TV équipé d'un mobile "MS-r4" à la fréquence de 60 Hz.

La composition selon l'invention comprend donc au moins une cire et au moins une huile, qui forment tout ou partie de la phase grasse. Le point de fusion finissant de ladite phase grasse totale est de préférence inférieur à 110°C, ce qui n'empêche pas que certains constituants puissent avoir un point de fusion supérieur.
On peut employer toute cire connue dans l'art antérieur et notamment les cires minérales telles que les cires microcristallines, la paraffine, le pétrolatum, la vaseline, l'ozokérite, la cire de montan; les cires animales telles que la cire d'abeilles, la lanoline et ses dérivés; les cires végétales telles que les cires de Candellila, d'Ouricoury, de Carnauba, du Japon, le beurre de cacao, les cires de fibres de lièges ou de canne à sucre; les huiles hydrogénés, les esters gras et les glycérides concrets à 25°C; les cires synthétiques, telles que les cires de polyéthylène et les cires obtenues par synthèse de Fischer-Tropsch; les cires de silicone. La phase grasse peut comprendre une cire ou un mélange de cires.
De préférence, la composition comprend 0,5-8% en poids de cire, notamment 1,5-6% en poids.
La phase grasse comprend également au moins une huile, ou un mélange d'huiles, usuellement utilisées en cosmétique, parmi lesquelles on peut citer les huiles minérales telles que l'huile de paraffine ou de vaseline; les huiles animales telles que le perhydrosqualène ou l'huile d'Arara; les huiles végétales telles que l'huile d'amande douce, de calophyllum, de palme, de ricin, d'avocat, de jojoba, d'olive ou de germes de céréales; les huiles de silicone; des esters d'acide lanolique, d'acide oléique, d'acide laurique, d'acide stéarique ou d'acide myristique par exemple; des alcools tels que l'alcool oléique, l'alcool linoléique ou linolénique, l'alcool isostéarique ou l'octyl dodécanol; des acétylglycérides, des octanoates, décanoates ou ricinoléates d'alcools ou de polyalcools.
La composition peut comprendre jusqu'à 93,3% en poids d'huile, de préférence entre 15 et 80% en poids.
La composition peut également comprendre d'autres corps gras tels que des composés hydrocarbonés pâteux, en faible quantité.
La phase grasse peut représenter jusqu'à 94% en poids de la composition, de préférence 70-90% en poids.

La composition comprend également au moins un agent épaississant, qui peut en particulier être choisi parmi les argiles telles que les bentonites ou les hectorites, éventuellement modifiées notamment par du chlorure de distéaryl diméthyl ammonium, ou par du chlorure de stéaryl diméthyl benzyl ammonium, ou encore par des silicates d'aluminium ou de magnésium. On peut également utiliser les dérivés d'huile de ricin hydrogénée, tels que le 'THIXINR' de Rheox.
L'agent épaississant, et/ou les charges, sont présents dans la composition de manière à épaissir au moins une partie des huiles, de préférence la totalité des huiles. On entend par là que le mélange 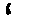 huile + charges + épaississant 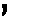 présente une viscosité de 50-250 Pa.s, de préférence 150-220 Pa.s, mesurée avec un viscosimètre rotatif CONTRAVES TV équipé d'un mobile "MS-r5" à la fréquence de 60 Hz.
L'agent épaississant peut être présent à raison de 0,2 à 10% en poids, de préférence à raison de 0,5 à 5% en poids de la composition.

La composition comprend également au moins 6% en poids, de préférence 8-25% en poids, de charges. Par charges, on entend dans la présente description des particules incolores ou blanches, minérales, organiques ou de synthèse, lamellaires ou sphériques, destinées à donner du corps ou de la rigidité à la composition.
Dans un mode de réalisation préféré de l'invention, lesdites charges sont principalement des charges sphériques, minérales et/ou organiques.

Les charges susceptibles d'absorber au moins une partie des huiles présentes dans la composition, peuvent être choisies parmi les charges minérales ou organiques usuelles, telles que le talc, le mica, la silice, le kaolin, les poudres de Nylon et de polyéthylène, l'amidon éventuellement réticulé, le nitrure de bore, les micro-sphères creuses telles que l'Expancel (Nobel Industrie), et les microbilles de résine de silicone (Tospearls de Toshiba, par exemple), et leurs mélanges.

La composition peut également comprendre des pigments et/ou des nacres, habituellement utilisés en cosmétique et qui peuvent être présents à raison de 0-20% en poids, de préférence à raison de 2-12%. Par pigments, il faut comprendre des particules blanches ou colorées, minérales ou organiques, destinées à colorer et/ou opacifier la composition. Par nacres, il faut comprendre des particules irisées, riches en calcaire produites par certains mollusques dans leur coquille.
Parmi ces pigments et/ou nacres, on peut citer les dioxydes de titane, de zirconium ou de cérium, ainsi que les oxydes de zinc, de fer ou de chrome, le bleu ferrique; le noir de carbone, et les laques de baryum, strontium, calcium, aluminium; le mica recouvert d'oxyde de titane ou d'oxychlorure de bismuth ainsi que le mica titane ou la nacre naturelle.

La composition peut comprendre en outre tout additif usuellement utilisé dans le domaine cosmétique tel que des antioxydants, des parfums, des huiles essentielles, des conservateurs, des actifs cosmétiques, des hydratants, des vitamines, des acides gras essentiels, des sphingolipides, des filtres solaires, des tensioactifs, des polymères liposolubles comme les polyalkylènes, les polyacrylates et les polymères siliconés compatibles avec les corps gras. Bien entendu l'homme du métier veillera à choisir ce/ces éventuels composés complémentaires, et/ou leur quantité, de manière telle que les propriétés avantageuses de la composition selon l'invention ne soient pas, ou substantiellement pas, altérées par l'adjonction envisagée.

Les compostions selon l'invention trouvent une application notamment dans le domaine du maquillage de la peau et/ou des semi-muqueuses et/ou des muqueuses, et peuvent se présenter par exemple sous la forme d'un fond de teint, d'un fard à joues ou à paupières, d'un rouge à lèvres, d'un mascara ou d'un eye-liner.
Elles peuvent également être utilisées comme base de soin pour les lèvres ou comme base fixante à appliquer sur un rouge à lèvres classique. La base fixante forme alors un film protecteur sur le film de rouge, en limite le transfert et la migration, et permet d'augmenter ainsi sa tenue.
Elles peuvent encore se présenter sous la forme d'un produit de soin de la peau, des muqueuses et/ou des semi-muqueuses, d'un produit hygiénique ou pharmaceutique ou encore d'un produit solaire ou autobronzant.

Un procédé de préparation de la composition selon l'invention consiste à préparer un prémélange d'au moins une partie des huiles avec au moins une partie de l'agent épaississant, à homogénéiser ledit prémélange puis à ajouter le reste des constituants. On a en effet constaté que, lors de la mise en oeuvre de ce procédé, les huiles liquides sont en quelque sorte 'piégées', lors de l'homogénéisation, dans une structure épaissie, voire gélifiée, d'où une composition résultante plus agréable à appliquer et à porter, et présentant une tenue améliorée, notamment sur les lèvres. De préférence, le prémélange comprend, avant homogénéisation, la totalité des huiles et/ou la totalité de l'agent épaississant.
L'homogénéisation peut être effectuée par tout moyen connu de l'homme du métier, en particulier par turbinage c'est-à-dire mélange à l'aide d'une turbine, ladite homogénéisation étant effectuée de manière à obtenir un prémélange épaissi. Après homogénéisation du prémélange, le reste des constituants, dont les charges, est ajouté, puis la composition résultante est mélangée, éventuellement chauffée et conditionnée.

L'invention est illustrée plus en détail dans les exemples suivants.

### Exemple 1

On prépare un rouge à lèvres sous forme de pâte molle ayant la composition suivante :

| | |
|---|---|
| . cires (abeilles et Carnauba) | 4 g |
| . huiles (ricin et octyldodécanol) | 16 g |
| . lanoline acétylée | 20 g |
| . lanoline | 30g |
| . épaississant (Bentone) | 1 g |
| . charges (amidon réticulé par l'anhydride octénylsuccinique, mica) | 20 g |
| . pigments | 9 g |

La composition est préparée de la manière suivante : on mélange dans un premier temps les huiles et l'épaississant, on homogénéise ledit mélange à l'aide d'une turbine de manière à obtenir un gel, puis l'on ajoute le reste des constituants et l'on chauffe jusqu'à environ 100°C en mélangeant de manière à obtenir un mélange parfaitement homogène.
Après refroidissement, on obtient un rouge à lèvres anhydre, facile à appliquer, et qui permet l'obtention d'un film brillant, agréable à porter, sans sensation de collant et de très bonne tenue.

### Exemple 2

On prépare un rouge à lèvres sous forme de pâte molle ayant la composition suivante :

| | |
|---|---|
| . cires (Carnauba et ozokérite) | 8 g |
| . huiles (ricin et huiles minérales) | 29 g |
| . lanoline | 40 g |
| . épaississant (Bentone) | 4 g |
| . charges (silice et talc) | 12 g |
| . pigments | 7 g |

La composition est préparée selon l'exemple 1. On obtient un rouge à lèvres anhydre, facile à appliquer, et qui permet l'obtention d'un film brillant, agréable à porter, sans sensation de collant.

### Exemple 3

On prépare un rouge à lèvres sous forme de pâte molle ayant la composition suivante :

| | |
|---|---|
| . cires (microcristalline et Candellila) | 6 g |
| . huiles (huile de sésame et palmitate d'octyle) | 48 g |
| . lanoline acétylée | 25,2 g |
| . épaississant (THIXINR de Rheox) | 0,8 g |
| . charges (silice, amidon et poudre de Nylon) | 15 g |
| . pigments | 5 g |

La composition est préparée selon l'exemple 1. On obtient un rouge à lèvres ayant de bonnes propriétés cosmétiques.

### Exemple 4 : Exemple comparatif

On prépare un rouge à lèvres sous forme de pâte molle ayant la composition suivante :

| | |
|---|---|
| . cires (Carnauba et ozokérite) | 6 g |
| . huiles (ricin et huiles minérales) | 64 g |
| . lanoline acétylée | 25 g |
| . pigments | 5 g |

La composition est préparée selon l'exemple 1.

On obtient un rouge à lèvres très huileux. Le film déposé sur les lèvres est de très mauvaise tenue et est très migrant.

### Exemple 5 : Exemple comparatif

On prépare un rouge à lèvres sous forme de pâte molle ayant la composition suivante :

| | |
|---|---|
| . cires (Carnauba et ozokérite) | 6 g |
| . huiles (ricin et huiles minérales) | 48 g |
| . lanoline acétylée | 41 g |
| . pigments | 5 g |

La composition est préparée selon l'exemple 1.

On obtient un rouge à lèvres très collant et très pâteux, d'application désagréable. Le film déposé sur les lèvres présente une certaine tendance à migrer.

## Revendications

1. Composition cosmétique se présentant sous forme de pâte souple, comprenant au moins une cire, au moins un agent épaississant, au moins une charge et au moins une huile, dans laquelle :
- ladite charge est présente à au moins 6% en poids dans la composition,
- ladite huile est épaissie par ledit agent épaississant et ladite charge, et le mélange 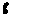 huile + charge + agent épaississant 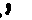 présente une viscosité de 50-250 Pa.s, et
- ladite composition finale présente une viscosité dynamique à 25°C de 3-30 Pa.s.

2. Composition selon la revendication 1, dans laquelle l'agent épaississant et/ou les charges sont présents en quantité telle que le mélange 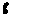 huile + charges + épaississant 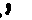 présente une viscosité de 150-220 Pa.s.

3. Composition selon l'une des revendications précédentes, comprenant 8-25 % en poids de charges.

4. Composition selon l'une des revendications précédentes, dans laquelle les charges sont des charges sphériques, minérales et/ou organiques.

5. Composition selon l'une des revendications 1 à 3, dans laquelle les charges sont choisies parmi le talc, le mica, la silice, le kaolin, les poudres de Nylon et de polyéthylène, l'amidon éventuellement réticulé, le nitrure de bore, les microsphères creuses et les microbilles de résine de silicone, et leurs mélanges.

6. Composition selon l'une des revendications précédentes, dans laquelle l'agent épaississant est présent à raison de 0,2-10% en poids, de préférence à raison de 0,5-5% en poids de la composition.

7. Composition selon l'une des revendications précédentes, dans laquelle l'agent épaississant est choisi parmi les argiles telles que les bentonites ou les hectorites, éventuellement modifiées notamment par du chlorure de distéaryl diméthyl ammonium, ou par du chlorure de stéaryl diméthyl benzyl ammonium ou par des silicates d'aluminium ou de magnésium; et/ou les dérivés d'huile de ricin hydrogénée.

8. Composition selon l'une des revendications précédentes, comprenant 0,5 à 8% en poids, de préférence 1,5-6% en poids, de cire.

9. Composition selon l'une des revendications précédentes, dans laquelle la cire est choisie parmi les cires minérales telles que les cires microcristallines, la paraffine, le pétrolatum, la vaseline, l'ozokérite, la cire de montan; les cires animales telles que la cire d'abeilles, la lanoline et ses dérivés; les cires végétales telles que les cires de Candellila, d'Ouricoury, de Carnauba, du Japon, le beurre de cacao, les cires de fibres de lièges ou de canne à sucre; les huiles hydrogénés, les esters gras et les glycérides concrets à 25°C; les cires synthétiques, telles que les cires de polyéthylène et les cires obtenues par synthèse de Fischer-Tropsch; les cires de silicone; et leurs mélanges.

10. Composition selon l'une des revendications précédentes, dans laquelle l'huile est présente en une quantité pouvant aller jusqu'à 93,3% en poids de la composition, de préférence 15-80% en poids.

11. Composition selon l'une des revendications précédentes, dans laquelle l'huile est choisie parmi les huiles minérales telles que l'huile de paraffine ou de vaseline; les huiles animales telles que le perhydrosqualène ou l'huile d'Arara; les huiles végétales telles que l'huile d'amande douce, de calophyllum, de palme, de ricin, d'avocat, de jojoba, d'olive ou de germes de céréales; les huiles de silicone; des esters d'acide lanolique, d'acide oléique, d'acide laurique, d'acide stéarique ou d'acide myristique par exemple; des alcools tels que l'alcool oléique, l'alcool linoléique ou linolénique, l'alcool isostéarique ou l'octyl dodécanol; des acétylglycérides, des octanoates, décanoates ou ricinoléates d'alcools ou de polyalcools; et leurs mélanges.

12. Composition selon l'une des revendications précédentes, se présentant sous la forme d'un produit de soin et/ou de maquillage de la peau et/ou des semi-muqueuses et/ou des muqueuses, notamment des lèvres du visage.

13. Composition selon l'une des revendications précédentes, se présentant sous la forme d'un fond de teint, d'un fard à joues ou à paupières, d'un rouge à lèvres, d'un mascara, d'un eye-liner, d'une base de soin pour les lèvres, d'une base fixante à appliquer sur un rouge à lèvres classique, d'un produit de soin, d'un produit hygiénique ou pharmaceutique, d'un produit solaire ou autobronzant.

14. Procédé de préparation d'une composition cosmétique se présentant sous forme de pâte souple, ayant une viscosité dynamique à 25°C de 3-30 Pa.s, comprenant au moins une cire, au moins un agent épaississant, au moins 6% en poids de charge et au moins une huile, dans lequel :
- on prépare un prémélange d'au moins une partie des huiles avec au moins une partie de l'agent épaississant,
- on homogénéise ledit prémélange,
- on ajoute le reste des constituants dont les charges, le mélange 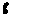 huile + charges + agent épaississant 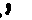 présentant une viscosité de 50-250 Pa.s.

15. Procédé selon la revendication 14, dans lequel on ajoute au prémélange, avant homogénéisation, la totalité des huiles et/ou la totalité de l'agent épaississant.

16. Composition cosmétique susceptible d'être obtenu par le procédé selon l'une des revendications 14 à 15.

17. Utilisation de la composition selon l'une des revendications 1-13 et 16 pour le maquillage et/ou le soin de la peau et/ou des semi-muqueuses et/ou des muqueuses, notamment des lèvres du visage.

18. Utilisation de la composition selon l'une des revendications 1-13 et 16 pour obtenir un film brillant et/ou de très bonne tenue et/ou qui ne transfère pas et/ou qui ne tache pas et/ou qui ne migre pas au cours du temps.

## Claims

1. Cosmetic composition in the form of a soft paste, comprising at least one wax, at least one thickener, at least one filler and at least one oil, in which:
- the said filler is present to at least 6% by weight in the composition,
- the said oil is thickened by the said thickener and the said filler, and the "oil + filler + thickener" mixture has a viscosity of 50-250 Pa.s, and
- the said final composition has a dynamic viscosity at 25°C of 3-30 Pa.s.

2. Composition according to Claim 1, in which the thickener and/or the fillers are present in an amount such that the "oil + fillers + thickener" mixture has a viscosity of 150-220 Pa.s.

3. Composition according to either of the preceding claims, comprising 8-25% by weight of fillers.

4. Composition according to one of the preceding claims, in which the fillers are inorganic and/or organic spherical fillers.

5. Composition according to one of Claims 1 to 3, in which the fillers are chosen from talc, mica, silica, kaolin, nylon powders, polyethylene powders, optionally crosslinked starch, boron nitride, hollow microspheres and microbeads of silicone resin, and mixtures thereof.

6. Composition according to one of the preceding claims, in which the thickener is present in a proportion of 0.2-10% by weight, preferably in a proportion of 0.5-5% by weight, of the composition.

7. Composition according to one of the preceding claims, in which the thickener is chosen from clays such as bentonites or hectorites, which are optionally modified, in particular with distearyldimethylammonium chloride or with stearyldimethylbenzylammonium chloride or with aluminium or magnesium silicates; and/or derivatives of hydrogenated castor oil.

8. Composition according to one of the preceding claims, comprising 0.5 to 8% by weight, preferably 1.5-6% by weight, of wax.

9. Composition according to one of the preceding claims, in which the wax is chosen from mineral waxes such as microcrystalline waxes, paraffin, petrolatum, petroleum jelly, ozokerite, montan wax; animal waxes such as beeswax, lanolin and derivatives thereof; plant waxes such as candelilla wax, ouricurry wax, carnauba wax, japan wax, cocoa butter, cork fibre wax or sugar cane wax; hydrogenated oils, fatty esters and glycerides that are solid at 25°C; synthetic waxes such as polyethylene waxes and the waxes obtained by Fischer-Tropsch synthesis; silicone waxes; and mixtures thereof.

10. Composition according to one of the preceding claims, in which the oil is present in an amount which may be up to 93.3% by weight of the composition, preferably 15-80% by weight.

11. Composition according to one of the preceding claims, in which the oil is chosen from mineral oils such as liquid paraffin or liquid petroleum jelly; animal oils such as perhydrosqualene or Arara oil; plant oils such as sweet almond oil, beauty-leaf oil, palm oil, castor oil, avocado oil, jojoba oil, olive oil or cereal germ oil; silicone oils; esters of lanolic acid, of oleic acid, of lauric acid, of stearic acid or of myristic acid, for example; alcohols such as oleyl alcohol, linoleyl alcohol or linolenyl alcohol, isostearyl alcohol or octyldodecanol; acetylglycerides, octanoates, decanoates or ricinoleates of alcohols or of polyalcohols; and mixtures thereof.

12. Composition according to one of the preceding claims, in the form of a care product and/or a make-up product for the skin and/or semi-mucous membranes and/or mucous membranes, in particular the lips of the face.

13. Composition according to one of the preceding claims, in the form of a foundation, a blusher, an eyeshadow, a lipstick, a mascara, an eye-liner, a care base for the lips, a fixing base to be applied to a standard lipstick, a care product, a hygiene product, a pharmaceutical product, an antisun product or a self-tanning product.

14. Process for the preparation of a cosmetic composition in the form of a soft paste, having a dynamic viscosity at 25°C of 3-30 Pa.s, comprising at least one wax, at least one thickener, at least 6% by weight of filler and at least one oil, in which
- a premix of at least some of the oils with at least some of the thickener is prepared,
- the said premix is homogenized,
- the remainder of the constituents are added, including the fillers, the "oil + fillers + thickener" mixture having a viscosity of 50-250 Pa.s.

15. Process according to Claim 14, in which all of the oils and/or all of the thickener is (are) added to the premix before homogenization.

16. Cosmetic composition which may be obtained by the process according to either of Claims 14 and 15.

17. Use of the composition according to one of Claims 1-13 and 16 to make up and/or care for the skin and/or semi-mucous membranes and/or mucous membranes, in particular the lips of the face.

18. Use of the composition according to one of Claims 1-13 and 16 in order to obtain a shiny film and/or a film with very good staying power and/or one which does not transfer and/or which does not leave marks and/or which does not migrate over time.

## Patentansprüche

1. Kosmetische Zusammensetzung, die in Form einer weichen Paste vorliegt und mindestens ein Wachs, mindestens ein Verdickungsmittel, mindestens einen Füllstoff und mindestens ein Öl enthält, worin:
- der Füllstoff in einem Mengenanteil von mindestens 6 Gew.-% in der Zusammensetzung vorliegt,
- das Öl durch das Verdickungsmittel und den Füllstoff verdickt ist, wobei das Gemisch "Öl + Füllstoff + Verdickungsmittel" eine Viskosität von 50-200 Pa.s aufweist, und
- die am Ende vorliegende Zusammensetzung eine dynamische Viskosität bei 25 °C von 3-30 Pa.s aufweist.

2. Zusammensetzung nach Anspruch 1, worin das Verdickungsmittel und/oder die Füllstoffe in einem solchen Mengenanteil vorliegen, daß das Gemisch "Öl + Füllstoffe + Verdickungsmittel" eine Viskosität von 150-220 Pa.s aufweist.

3. Zusammensetzung nach einem der vorhergehenden Ansprüche, die 8-25 Gew.-% Füllstoffe enthält.

4. Zusammensetzung nach einem der vorhergehenden Ansprüche, worin die Füllstoffe kugelförmige anorganische und/oder organische Füllstoffe sind.

5. Zusammensetzung nach einem der Ansprüche 1 bis 3, worin die Füllstoffe unter Talk, Glimmer, Siliciumdioxid, Kaolin, Nylonpulver, Polyethylenpulver, gegebenenfalls vernetzter Stärke, Bornitrid, Mikrohohlkugeln und Siliconharz-Mikrokugeln und deren Gemischen ausgewählt sind.

6. Zusammensetzung nach einem der vorhergehenden Ansprüche, worin das Verdickungsmittel in einem Mengenanteil von 0,2-10 Gew.-% und vorzugsweise von 0,5-5 Gew.-% der Zusammensetzung vorliegt.

7. Zusammensetzung nach einem der vorhergehenden Ansprüche, worin das Verdickungsmittel unter den Tonen, wie den Bentoniten oder Hectoriten, die gegebenenfalls insbesondere mit Distearyldimethylammoniumchlorid, Stearyldimethylbenzylammoniumchlorid oder Aluminium- oder Magnesiumsilicaten modifiziert sind, und/oder Derivaten von hydriertem Ricinusöl ausgewählt ist.

8. Zusammensetzung nach einem der vorhergehenden Ansprüche, die 0,5 bis 8 Gew.-% und vorzugsweise 1,5-6 Gew.-% Wachs enthält.

9. Zusammensetzung nach einem der vorhergehenden Ansprüche, worin das Wachs unter den Mineralwachsen, wie mikrokristallinen Wachsen, Paraffin, Petrolatum, Vaseline, Ozokerit und Montanwachs, tierischen Wachsen, wie Bienenwachs, Lanolin und seinen Derivaten, pflanzlichen Wachsen, wie Candellilawachs, Ouricuriwachs, Carnaubawachs, Japanwachs, Kakaobutter und Wachsen von Korkfasern oder Zuckerrohrfasern, hydrierten Ölen, Fettsäureestern und Glyceriden, die bei 25 °C fest werden, synthetischen Wachsen, wie Polyethylenwachsen und durch Fischer-Tropsch-Synthese hergestellten Wachsen, Siliconwachsen und deren Gemischen ausgewählt ist.

10. Zusammensetzung nach einem der vorhergehenden Ansprüche, worin das Öl in einem Mengenanteil von bis zu 93,3 Gew.-% der Zusammensetzung und vorzugsweise von 15-80 Gew.-% vorliegt.

11. Zusammensetzung nach einem der vorhergehenden Ansprüche, worin das Öl unter den Mineralölen, wie Paraffinöl oder Vaselineöl, tierischen Ölen, wie Perhydrosqualen oder Araraöl, pflanzlichen Ölen, wie Süßmandelöl, Calophyllumöl, Palmöl, Ricinusöl, Avocadoöl, Jojobaöl, Olivenöl oder Öl von Keimen von Cerealien, Siliconölen, Estern von Lanolinsäure, Ölsäure, Laurinsäure, Stearinsäure oder Myristinsäure, Alkoholen, wie Oleylalkohol, Linoleylalkohol, Linolenylalkohol, Isostearylalkohol oder Octyldodecanol, Acetylglyceriden, Octanoaten, Decanoaten oder Ricinoleaten von Alkoholen oder Polyalkoholen und deren Gemischen ausgewählt ist.

12. Zusammensetzung nach einem der vorhergehenden Ansprüche, die in Form eines Produkts zur Pflege und/oder zum Schminken der Haut und/oder der Semi-Schleimhäute und/oder der Schleimhäute, insbesondere der Lippen, vorliegt.

13. Zusammensetzung nach einem der vorhergehenden Ansprüche, die in Form von Make-up, Wangenrouge, Lidschatten, Lippenstift, Mascara, Eyeliner, Lippenpflegemittel, Mittel zur Fixierung, das über einem herkömmlichen Lippenstift aufgetragen wird, Pflegeprodukt, Produkt zur Hygiene oder pharmazeutisches Produkt, Sonnenschutzmittel oder Selbstbräunungsmittel vorliegt.

14. Verfahren zur Herstellung einer kosmetischen Zusammensetzung, die in Form einer weichen Paste vorliegt und eine dynamische Viskosität bei 25 °C von 3-30 Pa.s aufweist und mindestens ein Wachs, mindestens ein Verdickungsmittel, mindestens 6 Gew.-% Füllstoff und mindestens ein Öl enthält, wobei
- ein Vorgemisch aus mindestens einem Teil der Öle und mindestens einem Teil des Verdickungsmittels hergestellt wird,
- das Vorgemisch homogenisiert wird,
- die übrigen Bestandteile, und darunter die Füllstoffe, zugegeben werden, wobei das Gemisch "Öl + Füllstoffe + Verdickungsmittel" eine Viskosität von 50-250 Pa.s aufweist.

15. Verfahren nach Anspruch 14, worin vor der Homogenisierung die gesamten Öle und/oder das gesamte Verdickungsmittel zu dem Vorgemisch gegeben werden.

16. Kosmetische Zusammensetzung, die durch das Verfahren nach einem der Ansprüche 14 bis 15 hergestellt werden kann.

17. Verwendung einer Zusammensetzung nach einem der Ansprüche 1-13 und 16 zum Schminken und/oder zur Pflege der Haut und/oder der Semi-Schleimhäute und/oder der Schleimhäute, insbesondere der Lippen.

18. Verwendung der Zusammensetzung nach einem der Ansprüche 1-13 und 16 zur Herstellung eines glänzenden Films und/oder eines Films mit sehr guter Haftung und/oder eines Films, der sich nicht überträgt, und/oder eines Films, der nicht abfärbt, und/oder eines Films, der im Laufe der Zeit keine Migration aufweist.
